# FASCICULE DE BREVET EUROPEEN

(11) **EP 2 768 877 B1**
(45) Date de publication et mention de la délivrance du brevet: **13.09.2017**
(21) Numéro de dépôt: 12790592.5
(22) Date de dépôt: 18.10.2012
(51) Int. Cl.: C08G 18/28, C08G 18/64, C08G 18/76, C08G 18/78, C08G 18/79, C08G 18/80, C08L 95/00, C09D 195/00, C07C 271/06

(54) **PLASTIFIANT POLYMERISABLE, COMPOSITION DE RESINE POLYURETHANE LIQUIDE COMPRENANT LEDIT PLASTIFIANT ET SES UTILISATIONS**
POLYMERISIERBARE WEICHMACHER, FLÜSSIGE POLYURETHANHARZZUSAMMENSETZUNG DAMIT UND VERWENDUNGEN DAVON
POLYMERISABLE PLASTICISER, LIQUID POLYURETHANE RESIN COMPOSITION COMPRISING SAME AND USES THEREOF

(30) Priorité: 20.10.2011 FR 1159493
(43) Date de publication de la demande: 27.08.2014
(73) Titulaire: SOPREMA, 67100 Strasbourg (FR)
(72) Inventeur: DUROT, Louis, F-75012 Paris (FR); BINDSCHEDLER, Pierre-Etienne, F-67210 Obernai (FR); FRANCOIS BARSEGHIAN, Virginie, F-75013 Paris (FR); PERRIN, Rémi, F-67530 Boersch (FR)
(74) Mandataire: Cabinet Plasseraud
(86) Numéro de dépôt international: PCT/FR2012/052369
(87) Numéro de publication internationale: WO 2013/057429

(56) Documents cités:
- EP-A1- 1 108 735
- EP-A1- 1 798 248
- WO-A1-02/051901
- WO-A1-2010/106022
- US-A- 6 060 574

## Description

### Domaine d'application

La présente invention concerne un plastifiant polymérisable de basse viscosité, ayant un fort pouvoir diluant, ce qui permet d'abaisser ou de supprimer la teneur en solvant dans des compositions liquides de résine polyuréthane ou de résine polyuréthane bitumineuses tout en réduisant ou supprimant les inconvénients liés à l'utilisation de plastifiants exogènes (affaiblissement de la résistance mécanique, de la résistance au vieillissement, de l'adhérence, affaiblissement de la résistance à l'eau, etc.).

En outre, ledit plastifiant polymérisable permet d'améliorer la compatibilité avec des charges hydrocarbonées dans des compositions liquides de résine polyuréthane ou de résine polyuréthane bitumineuses destinées à être appliquées en couche mince afin de former un revêtement.

### Art antérieur

Les plastifiants sont fréquemment utilisés dans des compositions de résine polyuréthane, notamment dans des compositions de résine polyuréthane monocomposantes, pour élever l'extrait sec, abaisser la viscosité, réduire le bullage et éventuellement abaisser le coût de production. De plus, dans les compositions polymères composites, telles que notamment des compositions de résine polyuréthane bitumineuses, les huiles plastifiantes aromatiques et les plastifiants tels que le diisopropylnaphthalène, le phthalate de dioctyle (DOP), le phthalate de diisononyle (DINP), le Mesamoll®, le diisobutyrate de triméthylpentanediol (TXIB) et le phthalate de butylbenzyle, ont un rôle d'agent compatibilisant, qui permet d'avoir une composition homogène et stable.

De manière classique, les plastifiants sont exogènes et inertes, c'est-à-dire qu'ils ne réagissent pas sur eux-mêmes ou avec les polymères. En revanche, lors de leur adjonction, appelée plastification, les plastifiants exogènes écartent les chaînes polymères réduisant ainsi les interactions entre les chaînes. Néanmoins, les plastifiants exogènes et inertes présentent certains défauts car ils ont une influence sur le revêtement final. En effet, le plastifiant écarte les chaînes de polymères, ce qui conduit à un revêtement final ayant des propriétés mécaniques et de vieillissement affaiblies. De plus, l'écartement des chaînes de polymères donnera un revêtement susceptible d'absorber de l'eau, ce qui se traduira par un gonflement du revêtement et, à terme, un manque d'adhérence du revêtement. Or, si l'on veut obtenir un revêtement étanche, ce dernier ne doit pas présenter de défauts d'adhérence ni d'absorption d'eau importante. De surcroît, l'utilisation de plastifiant exogène et inerte ne permet pas d'obtenir une adhésivité suffisante pour assurer un accrochage suffisamment intime, résistant et durable sur des supports différents tels que le bitume ou le ciment, notamment sur des supports rugueux ou verticaux, et même sur des supports horizontaux avec eau stagnante sans primaire.

Il est ainsi souvent nécessaire, pour mettre en oeuvre ces compositions comprenant un plastifiant exogène, de prévoir un primaire d'adhérence et/ou une couche de protection spécifique supplémentaire.

La société Bayer, dans son brevet EP 1 108 735 divulgue une composition de résine polyuréthane non-bitumineuse bicomposante sans solvant. L'état liquide de la composition est obtenu par l'utilisation d'un plastifiant qui ne comporte pas de fonction isocyanate NCO ni de fonction hydroxyle (-OH), c'est-à-dire qu'il est non-polymérisable. Cependant, du fait de l'utilisation massive de ce plastifiant exogène, le revêtement final a des propriétés mécaniques réduites et une sensibilité accrue aux UV et aux parasites fongiques, tels que des moisissures et des champignons.

Une application importante des revêtements polyuréthanes est en extérieur. Lorsque l'on désire un revêtement coloré sur une zone circulable, on utilise une composition de résine polyuréthane sans bitume, ou résine polyuréthane non bitumineuse, qui présente une résistance mécanique élevée. En revanche, lorsque l'on souhaite réaliser un revêtement sur une zone non-circulable, notamment sur une toiture, on peut utiliser une composition de résine polyuréthane avec bitume, ou résine polyuréthane bitumineuse, qui présente une adhérence élevée sur support bitumineux ou sur support béton sans primaire. L'introduction de mélanges bitumineux permet de faire baisser le prix de la composition et d'améliorer l'adhérence sur un support bitumineux mais il est source de difficultés supplémentaires liées à la compatibilisation du bitume avec les résines polyuréthane polaires ou prépolymères polyuréthane polaires qui sont les plus couramment employés.

Actuellement, on ne trouve dans le commerce que des compositions de résine polyuréthane bitumineuse bicomposantes sans solvant ou alors des compositions de résine polyuréthane bitumineuse monocomposantes avec solvant qui intègrent des quantités importantes de plastifiants exogènes, généralement sous forme d'huiles aromatiques, et/ou de charge liquide ou de diluant liquide. Cependant, les revêtements obtenus avec de tels produits, ne sont pas applicables sur un support bitumineux. En effet, l'huile aromatique exsude, dilue le support et une couche liquide se forme entre le bitume de support et le bitume du revêtement d'étanchéité. Ainsi un tel revêtement n'est pas adapté à la réparation d'un revêtement bitumineux existant. De plus, les compositions de résine polyuréthane bitumineuses monocomposantes de l'art antérieur ne peuvent pas être utilisées à l'extérieur. En effet, après application, l'huile plastifiante s'évapore, générant, à relativement brève échéance, des micros-fissures dans le revêtement qui en affaiblissent les propriétés d'étanchéité. De telles résines polyuréthane bitumineuses utilisant une huile aromatique plastifiante comme agent compatibilisant, sont les produits usuels et courants du marché, qui ne peuvent pas être utilisés sans protection ou sur des supports bitumineux et qui ne s'emploient que sur des supports en béton ou en ciment avec protection.

Les produits décrits sous l'appellation "Tremco system" dans le brevet US 5 319 008 de la société Tremco, sont des produits qui, en plus des problèmes cités précédemment, présentent des viscosités comprises entre 40 000 et 75 000 centipoises. Il s'agit donc de produits qui doivent être chauffés avant application, communément appelés produits « hot-melt », car non liquides à température ambiante ou pas assez liquides à température ambiante pour être appliqués facilement. Ces produits ne sont donc pas viables commercialement.

Dans la demande de brevet FR 2 787 801, la demanderesse a décrit une composition de résine polyuréthane bitumineuse liquide, prête-à-l'emploi et stable. Cette composition est adaptée à l'étanchéification de surfaces extérieures auto-protégées. Cependant, pour être utilisable industriellement, cette composition doit contenir du solvant. Or les solvants sont indésirables, coutent chers et pour des raisons écologiques et de santé publique, leur utilisation est progressivement réduite et sera selon toute vraisemblance interdite dans quelques années.

La société Teroson, dans son brevet US 4 871 792, divulgue une composition de résine polyuréthane bitumineuse prête-à-l'emploi sans solvant. La compatibilisation entre le bitume et le prépolymère est réalisée par l'utilisation de deux plastifiants : une résine butyluréthane-formaldéhyde-carbamate et le 2,3-dibenzyltoluène. L'inconvénient de cette composition est qu'elle génère du formaldéhyde toxique, ce qui impose un étiquetage de toxicité particulier qui dissuade le consommateur. De plus, la composition a une consistance pâteuse plutôt que liquide ce qui lui confère une utilisation en tant que mastic et non comme un revêtement d'étanchéité liquide applicable à froid. Le mastic, étant très épais, n'a pas besoin d'avoir une aussi bonne résistance au vieillissement car seule une faible surface est exposée. Une telle composition, appliquée en tant que revêtement ayant une épaisseur de 1 à 3 millimètres, n'aurait pas une tenue suffisante en milieu extérieur et ne serait pas applicable à froid.

La société Tremco dans sa demande de brevet GB 2 242 435 divulgue une composition de résine polyuréthane bitumineuse sans solvant. L'état semi-liquide de la composition est dû à l'utilisation d'une molécule de type tensioactif ayant une tête semi-polaire et une chaîne aliphatique. Ce tensioactif est un bon agent compatibilisant qui disperse le bitume dans le prépolymère. Cependant ce tensioactif n'est pas un plastifiant ; un plastifiant doit donc nécessairement être ajouté en plus dans la composition si l'on veut que celle-ci soit liquide. Puisque la composition décrite ne contient pas de solvant, le plastifiant exogène, étant non-polymérisable, ne peut pas rester dans le revêtement final et l'on observe après application un phénomène d'exsudation important. Le document EP 1 798 248 Al concerne une composition de polyuréthane présentant d'excellentes propriétés d'adhésion et de résistance à l'eau. Les exemples 1 et 3 décrivent la réaction entre une mole d'un trimer HDI (fonctionnalité 3) et une mole de n-octadécanol ou d'alcool isostéarique. Les présents inventeurs ont trouvé qu'il était possible de pallier à tous les inconvénients liés à l'utilisation de plastifiant et/ou d'agent compatibilisant dans des compositions et produits de l'art antérieur tout en limitant, voir supprimant l'utilisation de solvant, en mettant en oeuvre un plastifiant polymérisable qui ne reste pas dans la composition sous sa forme libre après son application et qui permet la compatibilisation des bitumes avec des prépolymères.

Le plastifiant polymérisable selon l'invention permet d'abaisser la viscosité des compositions de résines polyuréthane, de préférence des compositions de résines polyuréthane monocomposantes, ce qui permet d'obtenir des compositions liquides de faible viscosité facilement applicables par les utilisateurs à la spatule, au rouleau ou à la brosse.

De plus, le plastifiant polymérisable selon l'invention permet de diminuer, voire de supprimer, l'utilisation de solvant dans des compositions de résines polyuréthane, de préférence des compositions de résines polyuréthane monocomposantes, ce qui limite, voire supprime les inconvénients liés à la présence de solvants et notamment :
- une odeur désagréable due aux composés organiques volatils,
- une toxicité donnant lieu à un étiquetage spécifique,
- des problèmes vis-à-vis des règlementations environnementales.

De plus, il a été constaté par la Demanderesse que les revêtements obtenus avec des compositions de résine polyuréthanes non-bitumineuses sans-solvant qui comprennent le plastifiant polymérisable adhèrent mieux à certaines supports que les revêtements obtenus avec des compositions avec solvant mais sans plastifiant polymérisable. Cette meilleure adhérence permet d'appliquer les compositions directement sur du béton, sans avoir besoin d'appliquer au préalable une couche de primaire d'adhérence.

De surcroit, le plastifiant polymérisable selon l'invention permet d'améliorer la compatibilité entre les charges bitumineuses et les prépolymères dans des compositions de résines polyuréthane bitumineuses, de préférence monocomposantes, ce qui permet de diminuer, voire de supprimer l'utilisation d'agents compatibilisants classiques tels que les plastifiants exogènes dans des compositions de résine polyuréthane bitumineuses. Ainsi les revêtements obtenus avec les plastifiants polymérisables selon l'invention n'ont pas les inconvénients suivants liés à l'utilisation de plastifiants exogènes classiques:
- affaiblissement de la résistance mécanique,
- affaiblissement de l'adhérence,
- un vieillissement réduit dans le temps,
- une absorption d'eau accrue.

De plus, le remplacement de plastifiants exogènes par le plastifiant polymérisable dans des compositions de résine polyuréthane bitumineuses supprime le phénomène d'exsudation desdits plastifiants exogènes, ce qui permet d'appliquer les compositions bitumineuses directement sur du bitume, alors que les compositions bitumineuses comprenant des plastifiants exogènes ne peuvent être appliquées que sur des supports en béton ou en ciment et peuvent de surcroît nécessiter l'application d'une couche de protection au dessus du revêtement bitumineux.

Ainsi, la présente invention porte sur un plastifiant polymérisable, sur des compositions de résine polyuréthane liquides, de préférence monocomposantes stables, le contenant et sur leur utilisation pour réaliser des revêtements d'étanchéité ou de protection dans lesquels ledit plastifiant n'est plus à l'état libre mais est polymérisé.

Les revêtements obtenus présentent de bonnes résistances mécaniques, ils résistent aux UV, au vieillissement par oxydation, à l'eau et aux agressions chimiques et ne présentent pas de défauts de surface ni de défaut d'adhérence (bulles, gonflement ou exsudation). De tels revêtements peuvent être circulables et sont particulièrement adaptés à une utilisation en milieu extérieur non-protégé comme revêtements d'étanchéité.

### Définitions

Dans la présente invention, par "composition liquide", on entend une composition ayant une viscosité comprise entre 1 000 et 40 000 centipoises, ladite viscosité étant mesurée à 23°C à l'aide d'un viscosimètre Brookfield (pour des viscosités inférieures à 10 000 centipoises, les mesures sont réalisées avec le module R5 à une vitesse de 30 tr/min et pour des viscosités supérieures à 10 000 centipoises, les mesures sont réalisées avec le module R6 à une vitesse de 20 tr/min). Une telle viscosité permet l'application de la composition notamment avec un rouleau communément appelé «rouleau à patte de lapin » ou une brosse pour former des couches de 0,5 à 2 mm d'épaisseur en une ou plusieurs applications.

Par "composition monocomposante ou composition prête-à-l'emploi" on entend une composition qui est destinée à être appliquée telle quelle par l'utilisateur final, c'est-à-dire par l'ouvrier qui va réaliser le revêtement d'étanchéité. Une telle composition prête-à-l'emploi est classiquement appelée dans la technique "monocomposante", par opposition aux compositions qui nécessitent l'ajout d'un catalyseur ou durcisseur ou autre agent réactif avant l'emploi ou qui doivent être mélangées pour une durée limitée (quelques heures) avant d'être appliquées.

Par "composition stable", on entend une composition qui peut-être stockée pendant un minimum de 4 mois sans observer de déphasage ou de prise en masse.

Par "revêtement circulable" on entend un revêtement qui présente une résistance mécanique suffisante pour permettre la circulation de personnes et de véhicules sur sa surface libre.

Par "revêtement ayant une bonne résistance mécanique", on entend un revêtement qui présente une résistance à la rupture supérieure ou égale à 2 MPa pour un revêtement non-circulable et une résistance à la rupture supérieure ou égale à 5 MPa pour un revêtement circulable (la résistance à la rupture est mesurée sur un appareil Instron selon la norme EN ISO 527-3). De façon classique, dans la pratique, pour un allongement supérieur à 100%, un revêtement non circulable présente une résistance à la rupture de 2 à 3 MPa et un revêtement circulable présente une résistance à la rupture de 5 à 8 MPa. Des valeurs supérieures pour un revêtement circulable sont évidemment acceptables.

Par "prépolymère" on entend un produit de réaction d'un polyol ou d'un mélange de polyols ayant un nombre de fonctions OH compris entre 1,5 et 3 et un poids moléculaire compris entre 900 et 3 000 g/mol, de préférence entre 1 000 et 2 800 g/mol et plus préférentiellement entre 1 500 et 2 500 g/mol avec un polyisocyanate ou un mélange de polyisocyanates ayant un nombre de fonction NCO compris entre 1,6 et 3 ; dans un ratio tel que le nombre de fonctions NCO du polyisocyanate ou du mélange de polyisocyanates par rapport au nombre de fonctions OH du polyol ou mélange de polyols soit de 1,5 à 2,5 environ.

Par "polyisocyanate" on entend un composé ayant plus d'une fonction isocyanate, le diisocyanate peut donc également être désigné dans la présente demande par polyisocyanate.

Par "TDI" on entend le toluène diisocyanate.

Par "MDI" on entend le diphénylméthane diisocyanate.

Par "HDI" on entend l'hexaméthylène diisocyanate.

Par "IPDI" on entend l'isophorone diisocyanate.

Par "nombre d'-OH de la molécule [A]" on entend le nombre de groupes OH présents sur la molécule [A].

Par "indice hydroxyle de [A]" on entend le nombre total de groupes hydroxyles réactifs sur [A], cette valeur pouvant être mesurée par dosage en retour avec la potasse. L'indice hydroxyle est exprimé en mg KOH/g ce qui correspond à la quantité de KOH en mg qui est nécessaire pour neutraliser 1 g de [A].

Par "solvant" on entend les solvants classiquement utilisés dans des compositions de résine polyuréthane, lesdits solvants étant inertes vis-à-vis des réactifs contenus dans la composition, liquides à température ambiante et présentant une température d'ébullition inférieure à 240°C.

Par "plastifiant exogène" on entend une molécule ou un oligomère ajouté aux compositions de résine polymérique, telle qu'une composition de résine polyuréthane, pour rendre le matériau résultant plus flexible, moins résistant, plus résilient et/ou plus facile à manipuler, ledit plastifiant exogène étant inerte c'est-à-dire qu'il ne comprend pas de fonctions réactives qui lui permettrait de réagir sur lui-même ou avec les prépolymères contenus dans la composition.

Par alkyle on entend un radical hydrocarbyle ayant 1 à 10 atomes de carbone, répondant à la formule générale CₙH₂ₙ₊₁ où n est supérieur ou égal à 1. Les groupes alkyles peuvent être linéaires ou ramifiés et peuvent être substitués par les groupements indiqués dans la présente demande.

Par aryle on entend un groupe hydrocarbyle polyinsaturé, aromatique, ayant un seul cycle (c'est-à-dire phényle) ou plusieurs cycles accolés (par exemple naphtyle) ou plusieurs cycles reliés par une liaison covalente (par exemple biphényle), qui contiennent typiquement 5 à 12 atomes de carbone, préférentiellement 6 à 10, et où au moins un cycle est aromatique. Le cycle aromatique peut optionnellement comprendre un à deux cycles supplémentaires (soit cycloalkyle, hétérocycloalkyle ou hétéroaryle) accolés. Le terme aryle comprend également les dérivés partiellement hydrogénés de systèmes carbocycliques décrits ci-dessus.

Lorsque les suffixes « ène » ou « diyl » sont employés en conjonction avec un groupement alkyle, cela veut dire que le groupe alkyle défini ci-dessus, présente deux liaisons simples comme point d'attache à d'autres groupes.

Par arylalkyle ou hétéroarylalkyle on entend un substituant alkyle linéaire ou ramifié ayant un atome de carbone attaché à un cycle aryle ou hétéroaryle.

Par hétéroaryle on entend un cycle ou deux cycles accolés ou reliés par une liaison covalente, comprenant 5 à 12 atomes de carbone, préférentiellement 6 à 10 atomes de carbone, où au moins l'un des cycles est aromatique et où au moins un ou plusieurs atomes de carbone sont remplacés par de l'oxygène, de l'azote et/ou du soufre. Le terme hétéroaryle comprend également des systèmes décrits ci-dessus ayant un groupe aryle, cycloalkyle, hétéroaryle ou hétérocycloalkyle accolé.

Par cycloalkyle on entend un hydrocarbyle monovalent, cyclique, insaturé ou saturé, présentant un ou 2 cycles et comportant 3 à 10 atomes de carbone.

Par hétérocycloalkyle on entend un cycloalkyle dans lequel au moins un atome de carbone est remplacé par un atome d'oxygène, d'azote et/ou de soufre.

Par arylcycloalkyle ou hétéroarylcycloalkyle on entend un cycloalkyle accolé ou lié par une liaison covalente à un cycle aryle ou hétéroaryle.

Par arylhétérocycloalkyle ou hétéroarylhétérocycloalkyle on entend un hétérocycloalkyle accolé ou lié par une liaison covalente à un cycle aryle ou hétéroaryle.

Par hydrocarbyle mono-ou polyinsaturé, on entend une chaîne hydrocarbonée ayant 2 à 30 atomes de carbones pouvant comporter au moins une insaturation.

Les groupes alkyle, aryle, arylalkyle, arylcycloalkyle, arylhétérocycloalkyle, hétéroaryl, hétéroarylalkyle, hydrocarbyle avec au moins une insaturation, chaîne hydrocarbonée mono ou polyinsaturée, peuvent en outre comporter un ou plusieurs substituants classiques choisis parmi : halogène, alkyle, cycloalkyle, hétérocycloalkyle, aryle, hétéroaryle, alcoxy, halogénoalkyle, arylalkyle, hétéroarylalkyle, arylhétérocycloalkyle.

### Plastifiant polymérisable

Un premier objet de l'invention est un plastifiant polymérisable constitué d'une chaîne hydrocarbonée dont une seule extrémité porte plus d'une fonction isocyanate, ladite chaîne hydrocarbonée comprenant et/ou étant substituée par un cycle aromatique et/ou un cycle aliphatique et/ou ladite chaîne hydrocarbonée est substituée par au moins deux chaînes hydrocarbonées pouvant comporter une insaturation, et le nombre de fonctions isocyanates étant strictement supérieur à 1, de préférence supérieur à 1,2, plus préférentiellement supérieur à 1,5 et inférieur ou égal à 2,2.

La chaîne hydrocarbonée dont une seule extrémité porte plus d'une fonction isocyanate ne doit pas être une chaîne purement aliphatique non substituée afin que le plastifiant polymérisable selon l'invention présente un pouvoir diluant suffisant pour remplacer une partie ou la totalité du solvant d'une composition liquide de résine polyuréthane.

De préférence, les deux chaînes hydrocarbonées pouvant comporter une insaturation qui peuvent substituer la chaîne hydrocarbonée dont une seule extrémité porte plus d'une fonction isocyanate ne comprennent pas d'atomes d'oxygène, de préférence elles ne comprennent pas d'autres atomes que le carbone et l'hydrogène.

Le nombre de fonctions isocyanates est estimé par calcul après titrage NCO par dosage en retour de l'excès de dibutylamine par de l'acide chlorhydrique (selon la norme EN ISO 14896-2006).

Le plastifiant polymérisable selon l'invention a un poids moléculaire compris entre 600 et 3 000 g/mol, de préférence entre 700 et 2 500 g/mol, et plus préférentiellement encore entre 800 et 2 000 g/mol.

Le plastifiant polymérisable selon l'invention doit présenter une viscosité la plus faible possible, étant entendu qu'il doit entrer dans des compositions liquides ayant une teneur limitée en, voire dépourvues de, solvant. Une viscosité acceptable, est comprise entre 400 et 14 000 centipoises, de préférence entre 1 300 et 13 000 centipoises et plus préférentiellement encore entre 2 000 et 12 000 centipoises, mesurée avec un viscosimètre Brookfield (mobile 6, vitesse 20 tr/min, 25°C). Dans le cas d'un plastifiant préparé à base de polyisocyanate aliphatique, la viscosité sera de l'ordre de 5 000 à 14 000 centipoises alors que dans le cas d'un plastifiant préparé à base de polyisocyanate aromatique, la viscosité sera plus faible, de l'ordre de 400 à 4 000 centipoises. Le pouvoir diluant et la faible viscosité du plastifiant polymérisable sont dus à la présence d'une chaîne hydrocarbonée qui n'est pas une chaîne purement aliphatique non-substituée et à un nombre de fonctions isocyanates inférieur ou égal à 2,2 présentes à une seule extrémité. En effet, si le plastifiant polymérisable comprenait des fonctions isocyanates à plus d'une extrémité, la viscosité serait trop élevée pour qu'il puisse être utilisé comme diluant en remplacement de tout ou partie du solvant contenu dans une composition de résine polyuréthane liquide. En outre, ce produit est un bon agent comptabilisant des prépolymères avec des mélanges bitumineux utilisant des bitumes naturels ou de synthèse grâce à sa chaîne hydrocarbonée portant ou substituée par un cycle aromatique et/ou un cycle aliphatique et/ou à sa chaîne hydrocarbonée substituée par au moins deux chaînes hydrocarbonées pouvant comporter une insaturation.

Le plastifiant polymérisable objet de l'invention, est destiné à entrer dans des compositions de résine polyuréthane ou de résine de polyuréthane bitumineuses liquides, de préférence monocomposantes stables, qui contiennent également des prépolymères. Après application de la composition sur une surface, le plastifiant polymérisable polymérise avec ces prépolymères. Contrairement à un plastifiant classique qui peut exsuder au vieillissement après application, le plastifiant polymérisable selon l'invention, n'exsudera pas car il ne se trouve plus à l'état libre dans le revêtement final. Les défauts liés à ce phénomène d'exsudation tels que la diminution de l'adhérence au support et des performances mécaniques, l'absorption d'eau, la mauvaise résistance au vieillissement, l'augmentation de la sensibilité aux UV et aux parasites fongiques, et l'apparition de bulles et de cloques sur le revêtement, seront donc évités par la mise en oeuvre du plastifiant polymérisable selon l'invention.

Selon un mode de réalisation particulier, le plastifiant polymérisable présente la formule générale (I) suivante : 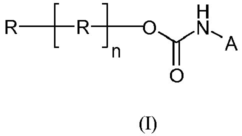 où chaque R représente indépendamment un motif qui ne comporte pas de fonction hydroxyle ni de fonction isocyanate et qui est choisi dans le groupe comprenant :
- alkyle,
- cycloalkyle,
- aryle,
- hétéroaryle,
- arylcycloalkyle,
- arylhétérocycloalkyle,
- hétéroarylalkyle,
- hétéroarylcycloalkyle,
- hydrocarbyle mono-ou polyinsaturée ;
et où n est compris entre 2 et 50, de préférence entre 3 et 30 et plus préférentiellement encore entre 5 et 25;
et où A est un groupe comportant un nombre de fonctions isocyanates strictement supérieur à 1, de préférence supérieur à 1,2, plus préférentiellement supérieur à 1,5 et inférieur ou égal à 2,2 ;
à la condition que la formule (I) comprenne soit au moins un cycle aromatique ou aliphatique dans au moins un motif R soit au moins deux motifs R alkyles substitués par une chaîne alkyle.

De préférence, le « groupe » de A est:
- une chaîne polymérique (provenant du MDI polymérique) répondant à la formule (A1) : 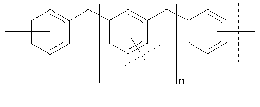 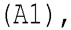
- un radical répondant à la formule (A2) ou à un radical apparenté d'un trimère de TDI : 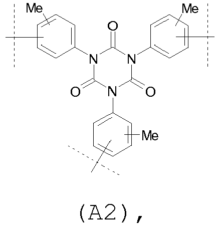
- un radical répondant à l'une des formules (A3) et/ou (A4) ou à un radical apparenté d'un trimère de HDI : 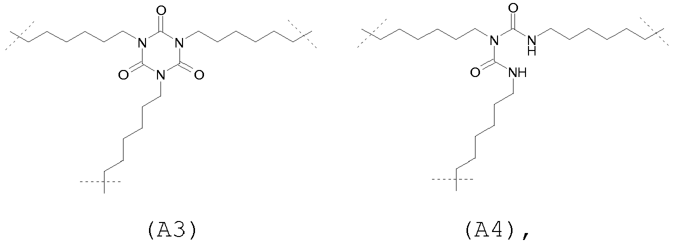
- un radical répondant à la formule (A5) ou à un radical apparenté d'un trimère d'IPDI : 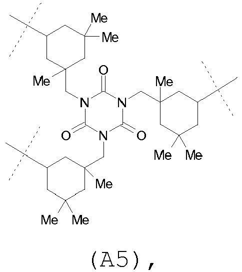 où chaque trait en pointillé représente un point d'attache à une fonction NCO ou au groupe -NHCOO-(R)ₙ-R de la formule (I) du plastifiant polymérisable et à la condition qu'il y ait au moins un trait en pointillé qui soit un point d'attache audit groupe -NHCOO-(R)ₙ-R et que les traits restants représentent un point d'attache à une fonction NCO.

Selon un mode de réalisation préférentiel, le plastifiant polymérisable présente la formule générale (II) suivante : 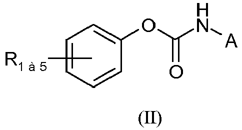 où les substituants R_{1 à 5} sont chacun indépendamment :
- halogène,
- alkyle,
- halogénoalkyle,
- cycloalkyle,
- aryle,
- alcoxy,
- arylalkyle,
- hétéroaryle,
- chaîne hydrocarbonée mono ou polyinsaturée;
et où R_{1 à 5} ne contiennent pas de fonction hydroxyle ni de fonction isocyanate ;
et où un au moins de R_{1 à 5} représente une chaîne polymère hydrocarbonée comprenant et/ou étant substituée par au moins un motif répétitif :
- alkyle,
- aryle,
- arylalkyle,
- arylcycloalkyle,
- arylhétérocycloalkyle,
- hétéroaryle,
- hétéroarylalkyle,
- hétéroarylcycloalkyle,
- hétéroarylhétérocycloalkyle,
- hydrocarbyle avec au moins une insaturation ;
et où A est un groupe comportant un nombre de fonctions isocyanates strictement supérieur à 1, de préférence supérieur à 1,2, plus préférentiellement supérieur à 1,5 et inférieur ou égal à 2,2.

Selon un mode de réalisation particulier, le plastifiant polymérisable présente la formule générale (II), où la chaîne polymère hydrocarbonée répond à la formule générale (IIIa) dans laquelle Z est un atome de carbone et/ou d'oxygène et n est compris entre 2 et 50, de préférence entre 3 et 30 et plus préférentiellement encore entre 5 et 25.

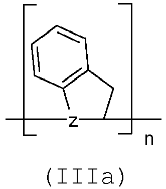

Selon un mode de réalisation particulier, le plastifiant polymérisable présente la formule générale (II), où la chaîne polymère hydrocarbonée répond à formule générale (IIIa) avec Z est un atome de carbone et/ou d'oxygène, où n est compris entre 2 et 50, de préférence entre 3 et 30 et plus préférentiellement encore entre 5 et 25, et où A est un radical répondant à la formule (A2) ou à un radical apparenté d'un trimère de TDI qui comporte un nombre de fonctions isocyanates strictement supérieur à 1 et inférieur ou égal à 2,2.

Selon un autre mode de réalisation, le plastifiant polymérisable présente la formule générale (II), où la chaîne polymère hydrocarbonée répond à formule générale (IIIa) avec Z est un atome de carbone et/ou d'oxygène, où n est compris entre 2 et 50, de préférence entre 3 et 30 et plus préférentiellement encore entre 5 et 25, et où A est un radical répondant à l'une des formules (A3) et/ou (A4) ou à un radical apparenté d'un trimère de HDI qui comporte un nombre de fonctions isocyanates strictement supérieur à 1 et inférieur ou égal à 2,2.

Selon un autre mode de réalisation, le plastifiant polymérisable présente la formule générale (II), où la chaîne polymère hydrocarbonée répond à formule générale (IIIa) avec Z est un atome de carbone et/ou d'oxygène, où n est compris entre 2 et 50, de préférence entre 3 et 30 et plus préférentiellement encore entre 5 et 25, et où A est un radical répondant à la formule (A5) ou à un radical apparenté d'un trimère d'IPDI qui comporte un nombre de fonctions isocyanates strictement supérieur à 1 et inférieur ou égal à 2,2.

Selon un mode de réalisation particulièrement préféré, le plastifiant polymérisable présente la formule générale (II), où la chaîne polymère hydrocarbonée répond à formule générale (IIIa) avec Z est un atome de carbone et/ou d'oxygène, où n est compris entre 2 et 50, de préférence entre 3 et 30 et plus préférentiellement encore entre 5 et 25, et où A est une chaîne polymérique (provenant du MDI polymérique) répondant à la formule (A1) qui comporte un nombre de fonctions isocyanates strictement supérieur à 1 et inférieur ou égal à 2,2.

Selon un mode de réalisation particulier, le plastifiant polymérisable présente la formule générale (II), où la chaîne polymère hydrocarbonée répond à la formule générale (IIIb) dans laquelle n est compris entre 2 et 50, de préférence entre 3 et 30 et plus préférentiellement encore entre 5 et 25.

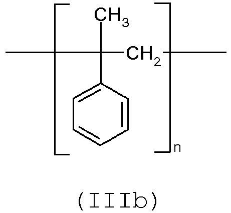

Selon un mode de réalisation particulier, le plastifiant polymérisable présente la formule générale (II), où la chaîne polymère hydrocarbonée répond à formule générale (IIIb), où n est compris entre 2 et 50, de préférence entre 3 et 30 et plus préférentiellement encore entre 5 et 25, et où A est un radical répondant à la formule (A2) ou à un radical apparenté d'un trimère de TDI qui comporte un nombre de fonctions isocyanates strictement supérieur à 1 et inférieur ou égal à 2,2.

Selon un autre mode de réalisation, le plastifiant polymérisable présente la formule générale (II), où la chaîne polymère hydrocarbonée répond à formule générale (IIIb), où n est compris entre 2 et 50, de préférence entre 3 et 30 et plus préférentiellement encore entre 5 et 25, et où A est un radical répondant à l'une des formules (A3) et/ou (A4) ou à un radical apparenté d'un trimère de HDI qui comporte un nombre de fonctions isocyanates strictement supérieur à 1 et inférieur ou égal à 2,2.

Selon un autre mode de réalisation, le plastifiant polymérisable présente la formule générale (II), où la chaîne polymère hydrocarbonée répond à formule générale (IIIb), où n est compris entre 2 et 50, de préférence entre 3 et 30 et plus préférentiellement encore entre 5 et 25, et où A est un radical répondant à la formule (A5) ou à un radical apparenté d'un trimère d'IPDI qui comporte un nombre de fonctions isocyanates strictement supérieur à 1 et inférieur ou égal à 2,2.

Selon un mode de réalisation particulièrement préféré, le plastifiant polymérisable présente la formule générale (II), où la chaîne polymère hydrocarbonée répond à formule générale (IIIb), où n est compris entre 2 et 50, de préférence entre 3 et 30 et plus préférentiellement encore entre 5 et 25, et où A est une chaîne polymérique (provenant du MDI polymérique) répondant à la formule (A1) qui comporte un nombre de fonctions isocyanates strictement supérieur à 1 et inférieur ou égal à 2,2.

Ledit plastifiant polymérisable selon l'invention est susceptible d'être obtenu par réaction entre:
- un composé de type alcool constitué d'une chaîne hydrocarbonée comprenant et/ou étant substituée par un cycle aromatique et/ou un cycle aliphatique et/ou la chaîne hydrocarbonée du composé de type alcool est substituée par au moins deux chaînes hydrocarbonées pouvant comporter une insaturation et où ledit composé de type alcool présente un nombre d'-OH compris entre 0,8% et 2,5%, de préférence entre 1,3% et 2,4% et plus préférentiellement encore entre 1,4% et 1,7% en poids par rapport au poids dudit composé et ;
- un polyisocyanate comprenant 2,1 à 3,2 fonctions isocyanates, de préférence 2,5 à 3,1 fonctions isocyanates.

De manière générale, le composé de type alcool est une résine ayant une seule fonction alcool, c'est à-dire une résine monohydroxylée également appelée monoalcool, de préférence une résine monohydroxylée phénolique également appelée monoalcool phénolique.

A titre d'exemple de résines monohydroxylées, on peut citer les résines terpéniques telles que α-pinène, β-pinène, dipentène, D-limonène et turpentine. A titre d'exemple de résines monohydroxylées phénoliques, on peut citer celles décrites dans Ullmanns Encyklopädie der technischen Chemie, 4ème édition, vol. 12, pages 539 à 545 (Verlag Chemie, Weinheim 1976) ; Kirk-Othmer, Encyclopedia of Chemical Technology, 3ème édition, vol. 12, pages 852 à 869 (John Wiley & Sons, New York, 1980) ; et Encyclopedia of Polymer Science and Engineering, vol. 7, pages 758 à 782 (John Wiley & Sons, New York, 1987).

Des exemples de monoalcools phénoliques préférées comprennent les résines alpha-méthylstyrène phénoliques et les résines de coumarone phénoliques.

Un exemple de composé de type alcool est un monoalcool phénolique de formule générale (IV) : 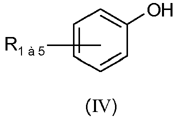 où R_{1 à 5} sont chacun indépendamment :
- halogène,
- alkyle,
- halogénoalkyle,
- cycloalkyle,
- aryle,
- alcoxy,
- arylalkyle,
- hétéroaryle,
- chaîne hydrocarbonée mono- ou polyinsaturée;
et où R_{1 à 5} ne contiennent pas de fonction hydroxyle ni de fonction isocyanate ;
et où un au moins de R_{1 à 5} représente une chaîne polymère hydrocarbonée comprenant et/ou étant substituée par au moins un motif répétitif :
- alkyle,
- aryle,
- arylalkyle,
- arylcycloalkyle,
- arylhétérocycloalkyle,
- hétéroaryle,
- hétéroarylalkyle,
- hétéroarylcycloalkyle,
- hétéroarylhétérocycloalkyle,
- hydrocarbyle avec au moins une insaturation.

Un autre exemple de monoalcool phénolique présente la formule générale (V) où chaque R représente indépendamment un arylhétérocycloalkyle et/ou un arylcycloalkyle comprenant 9 à 10 carbones et/ou un motif issu de la polymérisation de l'alpha-méthylstyrène.

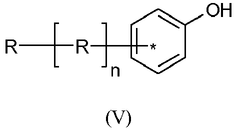

Un exemple particulier de résine phénolique est une résine selon la formule générale (V) où chaque R représente indépendamment benzofuranyle ou indényle.

Un exemple préféré de résine phénolique est une résine selon la formule générale (V) où chaque R représente un motif issu de la polymérisation de l'alpha-méthylstyrène.

Un exemple préféré de monoalcool phénolique est une résine de coumarone substituée par un phénol à une extrémité, telle que notamment la résine Novares® CA 100 ou la résine Novares® CA 120 commercialisées par la société RÜTGERS ou une résine alpha-méthylstyrène substituée par un phénol à une extrémité telle que notamment la résine Nevoxy® EPX-L5 commercialisée par la société NEVILLE, la résine Novares® LA 300 (CAS 68512-30-1) commercialisée par la société RÜTGERS ou la résine Epodil® L commercialisée par la société AIR PRODUCTS ou une résine hydrocarbonée aromatique phénolique telle que la résine Novares® LC 15 ou la résine Novares® LC 20 commercialisées par la société RÜTGERS.

Selon un mode de réalisation préférentiel, le monoalcool phénolique est la résine Novares® LA 300 (CAS 68512-30-1).

Le polyisocyanate peut notamment être un polyisocyanate aromatique, aliphatique ou cycloaliphatique.

Ledit polyisocyanate peut être choisi dans le groupe comprenant un trimère de TDI, un MDI polymérique, un trimère d'HDI, un trimère d'IPDI et leurs mélanges.

Un exemple de polyisocyanate aromatique utilisable est un diphénylméthane diisocyanate (MDI) polymérique ayant 2,7 fonctions isocyanates, tel que notamment le Suprasec® 5025 commercialisé par la société HUNTSMAN.

Un exemple de polyisocyanate aliphatique utilisable est un trimère d'HDI ayant plus ou moins 3 fonctions isocyanates, tel que notamment le Desmodur® N3300 ou le Desmodur® N100 commercialisés par la société BAYER.

### Composition de résine polyuréthane

Un second objet de l'invention est une composition de résine polyuréthane, de préférence une composition de résine polyuréthane monocomposante stable et liquide comprenant, outre un prépolymère, le plastifiant polymérisable décrit précédemment. Selon un mode de réalisation particulier, la composition de résine polyuréthane comprend le plastifiant polymérisable décrit précédemment, un prépolymère, et éventuellement un solvant, un catalyseur, des charges liquides et/ou des charges solides.

De préférence, la composition de résine polyuréthane selon l'invention n'est pas une composition de mousse de polyuréthane. De ce fait, selon un mode de réalisation particulier, la composition selon l'invention ne sera pas mélangée avec de l'eau pour polymériser et donner une mousse de polyuréthane. De la même façon la composition selon l'invention n'est pas destinée à être mélangée avec un agent de soufflage tel que notamment un gaz, par exemple le propane, butane, isobutane, dioxyde de carbone, monoxyde de carbone ou le diméthyléther pour former une mousse de polyuréthane.

Les prépolymères sont des produits commerciaux mais peuvent également être synthétisés avant de réaliser la composition de résine polyuréthane. De manière classique les prépolymères sont formés par réaction entre :
- un polyol ayant entre 1,5 et 3 fonctions OH et un poids moléculaire compris entre 900 et 3 000 g/mol, de préférence entre 1 000 et 2 800 g/mol et plus préférentiellement entre 1 500 et 2 500 g/mol ; et
- un di- et/ou polyisocyanate ayant entre 1,6 et 3 fonctions NCO ;
dans un ratio tel que le nombre de fonctions NCO du polyisocyanate par rapport au nombre de fonctions OH du polyol soit de 1,5 à 2,5 environ.

Le polyol utilisé pour former le prépolymère peut notamment être un polyol de type polyéther, polyester, polybutadiène, polycarbonate et leurs mélanges.

Le polyol de type polyéther peut notamment être un polypropylène glycol, un polyéthylène glycol, un triol de glycérine polypropylène glycol, un triol de glycérine polyéthylène glycol, ou un polytétrahydrofurane.

Le polyol de type polyester peut notamment être un polycaprolactone, un polyester d'acides gras dimères comprenant 34 à 36 atomes de carbone, un polyester polyadipate ou un polyester polyphthalate.

Le polyol polycarbonate peut notamment être un polycarbonate d'hexane-1,6-diol.

De préférence, le polyol ayant entre 1,5 et 3 fonctions OH et un poids moléculaire entre 900 et 3 000 g/mol entrant dans la formation du prépolymère est un polytétrahydrofurane, un polycarbonate d'hexane-1,6-diol, un polyester d'acides gras dimères comprenant 34 à 36 atomes de carbone, un polycaprolactone ou un polybutadiène hydroxylé.

Le di- et/ou polyisocyanate utilisé pour former le prépolymère peut notamment être le MDI, le MDI polymérique le TDI, un trimère de TDI, le HDI, une trimère de HDI, l'IPDI, un trimère d'IPDI, et leurs mélanges.

De préférence, le di- et/ou polyisocyanate entrant dans la formation du prépolymère est le MDI, le MDI polymérique le TDI, un trimère de TDI, et leurs mélanges.

Les charges qui peuvent être introduites dans la présente composition sont notamment des charges liquides qui sont les plastifiants exogènes, des bitumes naturels ou synthétiques ou un mélange bitumineux liquide aussi appelé « cut-back », et des charges solides telles que pigments, carbonate de calcium, oxyde de titane, ou autres.

Le catalyseur qui peut être introduit dans la présente composition permet d'accélérer la polymérisation de la composition. Les catalyseurs pouvant entrer dans la composition selon l'invention sont des catalyseurs classiquement utilisés dans des compositions de polyuréthane. A titre d'exemple, on peut citer les catalyseurs organométalliques à base de bismuth, de vanadium ou d'étain, tels que le dilaurate de dibutylétain, des amines tertiaires, telles que Jeffcat® DMDLS commercialisée par la société HUNSTMAN.

La présente composition contient peu ou pas de solvant. Par peu de solvant on entend une teneur en solvant inférieure à 10%, de préférence inférieure à 5%, et plus préférentiellement inférieure à 2% en poids par rapport au poids de la composition. En effet, le plastifiant polymérisable objet de l'invention a un fort pouvoir abaisseur de viscosité. Ainsi les compositions réalisées avec ledit plastifiant polymérisable sont liquides même si elles ne contiennent que peu ou pas de solvant. Cet avantage est un atout économique et écologique considérable.

Selon un mode de réalisation particulièrement préféré, les compositions ne contiennent pas du tout de solvant.

Une composition selon l'invention comprend:
- de 30 à 90%, de préférence de 35 à 75% et plus préférentiellement encore de 40 à 60% en poids d'un prépolymère par rapport au poids de la composition;
- de 10 à 70%, de préférence de 15 à 50% et plus préférentiellement encore de 20 à 45% en poids de plastifiant polymérisable par rapport au poids de la composition;
- de 0 à 20%, de préférence de 2 à 15% et plus préférentiellement encore de 5 à 10% en poids d'une charge solide par rapport au poids de la composition;
- de 0 à 60%, de préférence de 5 à 40% et plus préférentiellement encore de 10 à 35% en poids d'une charge liquide par rapport au poids de la composition;
- de 0 à 10%, de préférence de 0 à 5% et plus préférentiellement encore de 0 à 2% en poids de solvant par rapport au poids de la composition;
- de 0 à 5%, de préférence de 0,5 à 3 % et plus préférentiellement de 1,2 à 2% en poids de catalyseur par rapport au poids de la composition.

### Méthodes

La présente invention a également pour objet une méthode pour diminuer la quantité de solvant dans des compositions de résine polyuréthane ou dans des compositions de résine polyuréthane bitumineuse, de préférence monocomposantes. Cette méthode consiste à ajouter le plastifiant polymérisable selon l'invention aux constituants d'une composition de résine polyuréthane ou d'une composition de résine polyuréthane bitumineuse classique, en remplacement d'une partie ou de la totalité du solvant et/ou des plastifiants exogènes classiquement utilisés.

La méthode selon l'invention permet d'abaisser la teneur en solvant en dessous de 10%, de préférence en dessous de 5% et plus préférentiellement encore en dessous de 2%. Dans un mode de réalisation préféré, elle permet de supprimer la totalité du solvant.

La présente invention a également pour objet une méthode pour diminuer, voire supprimer, la quantité de plastifiant exogène dans des compositions de résine polyuréthane ou dans des compositions de résine polyuréthane bitumineuse, de préférence monocomposantes. Cette méthode consiste à ajouter le plastifiant polymérisable selon l'invention aux constituants d'une composition de résine polyuréthane ou d'une composition de résine polyuréthane bitumineuse classique, en remplacement d'une partie ou de la totalité du (des) plastifiant (s) exogène(s) classiquement utilisé(s).

La présence du plastifiant polymérisable selon l'invention permet également d'incorporer des bitumes naturels ou synthétiques dans une composition de polyuréthane qui ne contient pas d'agents compatibilisants tels que des plastifiants exogènes, et permet d'obtenir une composition liquide et stable.

### Utilisation

Un autre objet de l'invention est l'utilisation du plastifiant polymérisable tel que décrit précédemment dans des compositions de résine polyuréthane, de préférence dans des compositions de résine polyuréthane monocomposantes stables. L'utilisation du plastifiant polymérisable selon l'invention permet d'abaisser la viscosité de la composition et améliore la compatibilisation entre les charges hydrocarbonées et les prépolymères, ce qui permet d'obtenir une composition stable.

L'invention porte également sur l'utilisation de la composition selon l'invention pour réaliser un revêtement, notamment un revêtement d'étanchéité, présentant de bonnes résistances mécaniques, résistant aux UV, au vieillissement par oxydation, à l'eau et aux agressions chimiques et ne présentant pas de défauts de surface ni de défaut d'adhérence (bulles, gonflement ou exsudation). De tels revêtements peuvent être circulables et sont particulièrement adaptés à une utilisation en milieu extérieur non-protégé comme revêtements d'étanchéité. Les revêtements obtenus présentent une reprise en eau tout à fait satisfaisante, c'est-à-dire inférieure à 8% après 28 jours d'immersion dans de l'eau à 20°C. Les revêtements obtenus par l'utilisation de la composition selon l'invention peuvent recouvrir des surfaces horizontales, obliques, verticales, rugueuses et/ou comportant des points singuliers.

Les compositions de résine polyuréthane non-bitumineuses sont préférentiellement utilisées pour étanchéifier des surfaces horizontales circulables en extérieur, telles que des balcons, des gradins de stade, des parkings, des cours d'immeuble, etc...

Les compositions de résine polyuréthane bitumineuses sont préférentiellement utilisées pour réaliser des relevés, c'est-à dire pour réaliser un revêtement étanche entre une surface bitumineuse et un mur vertical ou un point singulier ou encore pour rénover des toitures.

L'invention va être décrite plus en détails à l'aide des exemples suivants qui sont donnés à titre purement illustratif.

### EXEMPLES

Dans les exemples, les parts sont exprimées en poids. Les viscosités sont mesurées à l'aide d'un viscosimètre Brookfield, mobile 5 ou 6, vitesse 20 tr/min à 23°C, moins d'une semaine après la fabrication du produit ou de la composition.

Dans les exemples, les produits commerciaux et les acronymes suivants sont utilisés:
Novares® LA 300 : résine d'alpha-méthylstyrène terminée par un phénol (CAS 68512-30-1) ayant un nombre d'-OH entre 1,7 et 2,2 commercialisée par la société RÜTGERS.
Suprasec® 5025 : MDI polymérique ayant 2,7 fonctions isocyanates commercialisé par la société HUNTSMAN.
Suprasec® 2385 : MDI modifié ayant 2 fonctions isocyanates commercialisé par la société HUNTSMAN.
Desmodur® N3300 : trimère de HDI ayant 3 fonctions isocyanates commercialisé par la société BAYER.
Desmodur® L 75 : trimère de TDI ayant 3 fonctions isocyanates commercialisé par la société BAYER.
Desmodur® Z 4470 : trimère d'IPDI ayant 3 fonctions isocyanates, en solution à 70% dans l'acétate de butyle ou un solvant naphta, commercialisé par la société BAYER.
Voranol® 2000 : polypropylène glycol ayant un poids moléculaire de 2000 g/mol (CAS 025322-69-4) commercialisé par la société DOW CHEMICAL.
Voranol® CP 450 : triol de glycérine polypropylène glycol (CAS 025791-96-2) ayant un poids moléculaire de 450 g/mol commercialisé par la société DOW CHEMICAL.
Terathane® 2000 : polytétrahydrofurane ayant un poids moléculaire de 2000 g/mol (CAS 24979-97-3) commercialisé par la société DUPONT.
Voranate® T80 : TDI commercialisé par la société DOW CHEMICAL.
Ruetasolv® Di: huile aromatique plastifiante de diisopropylnaphthalène commercialisée par la société RÜTGERS.
Incozol® LV : bis-oxazolidine comprenant un groupe carbonate (CAS 145899-78-1) commercialisée par la société INCOREZ.
PCP 1000 : polycaprolactone ayant 2 fonctions OH et un poids moléculaire de 1 000 g/mol commercialisé par la société SOLVAY.

### EXEMPLE 1: préparation d'un plastifiant polymérisable

Dans un réacteur on mélange les constituants suivants :
100 parts de résine de Novares® LA 300,
25 parts de Suprasec® 5025.

On chauffe le mélange au moins 12 heures aux environs de 100°C.

On obtient un plastifiant polymérisable ayant une viscosité de 1 300 centipoises et un poids moléculaire de 1 600 g/mol.

### EXEMPLE 2: préparation d'un plastifiant polymérisable

Dans un réacteur on mélange les constituants suivants :
100 parts de Novares® LA 300,
36 parts de Suprasec® 5025.

On chauffe le mélange au moins 12 heures aux environs de 100°C.

On obtient un plastifiant polymérisable ayant une viscosité de 1 500 centipoises et un poids moléculaire de 1 300 g/mol.

### EXEMPLE 3 : préparation d'un plastifiant polymérisable

Dans un réacteur on mélange les constituants suivants :
100 parts de Novares® LA 300,
36 parts de Desmodur® N3300.

On chauffe le mélange au moins 12 heures aux environs de 100°C.

On obtient un plastifiant polymérisable ayant une viscosité de 9 000 centipoises et un poids moléculaire de 1 700 g/mol.

### EXEMPLE 4: préparation d'un plastifiant polymérisable

On procède comme dans l'exemple 2, en modifiant les quantités de la façon suivante:
100 parts de Novares® LA 300
38 parts de Suprasec® 5025.

On obtient un plastifiant polymérisable ayant une viscosité d'environ 1 500 centipoises et un poids moléculaire de 2 200 g/mol.

### EXEMPLE 5: préparation d'un plastifiant polymérisable

On procède comme dans l'exemple 2, en modifiant les quantités de la façon suivante:
100 parts de Novares® LA 300
60 parts de Desmodur® L 75.

On obtient un plastifiant polymérisable ayant une viscosité d'environ 1 200 centipoises et un poids moléculaire de 1 659 g/mol.

### EXEMPLE 6: préparation d'un plastifiant polymérisable

On procède comme dans l'exemple 2, en modifiant les quantités de la façon suivante:
100 parts de Novares® LA 300
60 parts de Desmodur® Z 4470.

On obtient un plastifiant polymérisable ayant une viscosité d'environ 700 centipoises et un poids moléculaire de 1 666 g/mol.

### EXEMPLE 7: préparation d'un plastifiant polymérisable

On procède comme dans l'exemple 2, en modifiant les quantités de la façon suivante:
100 parts de Novares® LA 300
40 parts de Desmodur® N 3300.

On obtient un plastifiant polymérisable ayant une viscosité d'environ 2 500 centipoises et un poids moléculaire de 1 400 g/mol.

### EXEMPLE 8 : préparation d'une composition de résine polyuréthane liquide (comparatif)

Dans un réacteur on mélange les ingrédients suivants afin de former le prépolymère :
200 parts Voranol® 2000,
24 parts de Voranol® CP 450,
60 parts de Voranate® T80.

On agite le mélange à 80°C pendant 2 heures puis on après refroidissement vers 40°C on ajoute les constituants suivants :
24 parts de xylène,
150 parts Ruetasolv® Di,
200 parts de carbonate de calcium en tant que charges pulvérulentes.

La composition obtenue présente une viscosité Brookfield mobile 5, vitesse 10 à 23°C de 1 800 centipoises et est appliquée en tant que revêtement en intérieur sous un carrelage. Cette composition dégage une forte odeur de solvant et le revêtement obtenu présente une importante absorption d'eau d'environ 17% et un manque d'adhérence due à l'utilisation d'huile plastifiante.

### EXEMPLE 9 : préparation d'une composition de résine polyuréthane liquide comprenant le plastifiant polymérisable

Dans un réacteur on mélange les ingrédients suivants afin de former le prépolymère :
200 parts de Voranol® 2000,
24 parts de Voranol® CP 450,
60 parts de Voranate® T80.

On agite le mélange à 80°C pendant 2 heures puis on ajoute les constituants suivants :
200 parts de plastifiant polymérisable préparé selon l'exemple 4,
200 parts de charges pulvérulentes.

On obtient une composition monocomposante liquide sans solvant qui présente une viscosité Brookfield mobile 5, vitesse 10 à 23°C de 4 500 centipoises. La composition est appliquée en tant que revêtement en intérieur sous un carrelage. Le revêtement obtenu présente une absorption d'eau réduite aux environs de 7% et adhère parfaitement au support béton.

La composition est stockée pendant 4 mois à 20°C. Après 4 mois, la composition devient rapidement homogène lorsqu'on la mélange avec un bâton, aucun déphasage n'est observé.

### EXEMPLE 10 : préparation d'une composition de résine polyuréthane liquide comprenant le plastifiant polymérisable.

Dans un réacteur on mélange les ingrédients suivants afin de former un MDI masqué par l'hexanol :
37 parts de Suprasec® 5025,
10,2 parts d'hexanol.

L'hexanol est progressivement ajouté au MDI afin de maintenir la température du mélange réactionnel inférieure à 60°C. Lorsque l'ajout d'hexanol est terminé, on laisse le mélange revenir à la température ambiante.

Dans un réacteur on mélange les ingrédients suivants afin de former le prépolymère :
24 parts de Terathane® 2000,
6 parts de Suprasec® 2385,
6 parts de MDI masqué par l'hexanol synthétisé ci-dessus,
9 parts de Ruetasolv® Di,
0,084 parts de chlorure de benzoyle.

Le mélange réactionnel est agité à 400 tr/min pendant 3h à 50°C.

Dans une cuve à dispersion, on mélange les ingrédients suivants afin de former la composition de résine polyuréthane :
48,3 parts de prépolymère synthétisé ci-dessus,
38,7 parts de plastifiant polymérisable synthétisé selon l'exemple 1,
4,3 parts de pigment gris RAL 7040,
5,9 parts d'Incozol® LV,
2,4 parts d'anhydride méthyltétrahydrophtalique,
0,4 parts de chlorure de benzoyle.

On agite le mélange à température ambiante à une vitesse de 600 tr/min pendant 6 minutes.

On obtient une composition monocomposante liquide sans solvant ayant une viscosité Brookfield mobile 6, vitesse 20 à 23°C de 14 000 centipoises. La composition est appliquée directement sur du béton en tant que revêtement en intérieur sous un carrelage. Le revêtement obtenu présente une absorption d'eau réduite aux environs de 7% et adhère parfaitement au support béton.

La composition est stockée pendant 4 mois à 20°C. Après 4 mois, la composition devient rapidement homogène lorsqu'on la mélange avec un bâton, aucun déphasage n'est observé.

### EXEMPLE 11 : préparation d'une composition de résine polyuréthane liquide comprenant le plastifiant polymérisable

Dans un réacteur on mélange les ingrédients suivants afin de former le prépolymère :
540 parts de Voranate® T80,
90 parts de butane-1,4-diol,
1000 parts de PCP 1000,
45 parts de Voranol® CP 450,
120 parts d'acétate de butyle.

On agite le mélange à 80°C pendant 1h30.

Dans une cuve à dispersion, on mélange les ingrédients suivants afin de former la composition de résine polyuréthane :
100 parts de prépolymère synthétisé ci-dessus,
50 parts de plastifiant polymérisable synthétisé selon l'exemple 7,
10 parts de pigment gris RAL 7040,
14 parts d'Incozol® LV,
1 part d'anhydride méthyltétrahydrophtalique,

On agite le mélange à température ambiante à une vitesse de 600 tr/min pendant 6 minutes.

On obtient une composition monocomposante liquide à teneur réduite en solvant (4% de solvant) ayant une viscosité Brookfield mobile 6, vitesse 20 à 23°C de 6 000 centipoises. La composition est appliquée directement sur du béton en extérieur, par exemple sur un balcon. Le revêtement obtenu présente une absorption d'eau réduite aux environs de 7% et adhère parfaitement sur certains supports béton.

## Revendications

1. Plastifiant polymérisable constitué d'une chaîne hydrocarbonée dont une seule extrémité porte plus d'une fonction isocyanate, ladite chaîne hydrocarbonée comprenant et/ou étant substituée par un cycle aromatique et/ou un cycle aliphatique et/ou ladite chaîne hydrocarbonée est substituée par au moins deux chaînes hydrocarbonées pouvant comporter une insaturation, et le nombre de fonctions isocyanates étant strictement supérieur à 1, de préférence supérieur à 1,2, plus préférentiellement supérieur à 1,5 et inférieur ou égal à 2,2.

2. Plastifiant polymérisable selon la revendication 1, présentant un poids moléculaire compris entre 600 et 3 000 g/mol, de préférence entre 700 et 2 500 g/mol, et plus préférentiellement encore entre 800 et 2 000 g/mol.

3. Plastifiant polymérisable selon la revendication 1 ou 2, présentant une viscosité comprise entre 400 et 14 000 centipoises, de préférence entre 1 300 et 13 000 centipoises, et plus préférentiellement encore entre 2 000 et 12 000 centipoises.

4. Plastifiant polymérisable selon l'une quelconque des revendications 1 à 3, présentant la formule générale (I) : 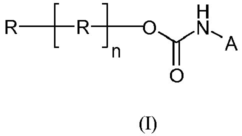 où chaque R représente indépendamment un motif répétitif qui ne comporte pas de fonction hydroxyle ni de fonction isocyanate et qui est choisi dans le groupe comprenant :
- alkyle,
- cycloalkyle,
- aryle,
- hétéroaryle,
- arylcycloalkyle,
- arylhétérocycloalkyle,
- hétéroarylalkyle,
- hétéroarylcycloalkyle,
- hydrocarbyle mono- ou polyinsaturé;
et où n est compris entre 2 et 50, de préférence entre 3 et 30 et plus préférentiellement encore entre 5 et 25;
et où A est un groupe comportant un nombre de fonctions isocyanates strictement supérieur à 1, de préférence supérieur à 1,2, plus préférentiellement supérieur à 1,5 et inférieur ou égal à 2,2 ;
à la condition que la formule (I) comprenne soit au moins un cycle aromatique ou aliphatique dans au moins un motif R soit au moins deux motifs R alkyles substitués par une chaîne alkyle.

5. Plastifiant polymérisable selon l'une quelconque des revendications 1 à 4, présentant la formule générale (II) 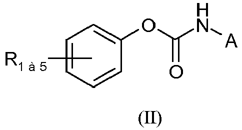 où les substituants R_{1 à 5} sont chacun indépendamment :
- halogène,
- alkyle,
- halogénoalkyle,
- cycloalkyle,
- aryle,
- alcoxy,
- arylalkyle,
- hétéroaryle,
- chaîne hydrocarbonée mono ou polyinsaturée;
et où R_{1 à 5} ne contiennent pas de fonction hydroxyle ni de fonction isocyanate; et où un au moins de R_{1 à 5} représente une chaîne polymère hydrocarbonée comprenant et/ou étant substituée par au moins un motif répétitif :
- alkyle,
- aryle,
- arylalkyle,
- arylcycloalkyle,
- arylhétérocycloalkyle,
- hétéroaryle,
- hétéroarylalkyle,
- hétéroarylcycloalkyle,
- hétéroarylhétérocycloalkyle,
- hydrocarbyle avec au moins une insaturation ;
et où A est un groupe comportant un nombre de fonctions isocyanates strictement supérieur à 1, de préférence supérieur à 1,2, plus préférentiellement supérieur à 1,5 et inférieur ou égal à 2,2.

6. Plastifiant polymérisable selon la revendication 5, **caractérisé en ce que** la chaîne polymère hydrocarbonée répond à la formule générale (IIIa) ou (IIIb) : 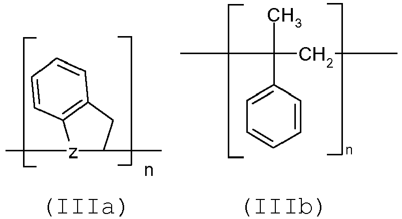 où Z est un atome de carbone et/ou d'oxygène ; et où n est compris entre 2 et 50, de préférence entre 3 et 30 et plus préférentiellement encore entre 5 et 25.

7. Plastifiant polymérisable selon la revendication 6, **caractérisé en ce que** la chaîne polymère hydrocarbonée répond à formule générale (IIIb) où n est compris entre 2 et 50, de préférence entre 3 et 30 et plus préférentiellement encore entre 5 et 25, et où A est une chaîne polymérique provenant du MDI polymérique répondant à la formule (A1) : 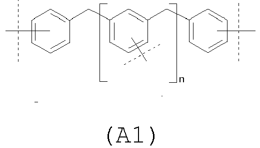 et où A comporte un nombre de fonctions isocyanates strictement supérieur à 1 et inférieur ou égal à 2,2.

8. Plastifiant polymérisable selon la revendication 6, **caractérisé en ce que** la chaîne polymère hydrocarbonée répond à formule générale (IIIb), où n est compris entre 2 et 50, de préférence entre 3 et 30 et plus préférentiellement encore entre 5 et 25, et où A est un radical répondant à la formule (A2) ou à un radical apparenté d'un trimère de TDI : 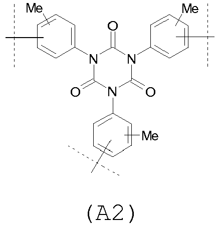 et où A comporte un nombre de fonctions isocyanates strictement supérieur à 1 et inférieur ou égal à 2,2.

9. Plastifiant polymérisable selon l'une quelconque des revendications 1 à 8, **caractérisé en ce qu'**il est susceptible d'être obtenu par la réaction entre :
- un composé de type alcool constitué d'une chaîne hydrocarbonée comprenant et/ou étant substituée par un cycle aromatique et/ou un cycle aliphatique et/ou la chaîne hydrocarbonée du composé de type alcool est substituée par au moins deux chaînes hydrocarbonées pouvant comporter une insaturation et où le composé de type alcool présente un nombre d'-OH compris entre 0,8% et 2, 5% , de préférence entre 1,3% et 2,4% et plus préférentiellement encore entre 1,4% et 1,7% en poids par rapport au poids dudit composé et ;
- un polyisocyanate comprenant 2,1 à 3,2 fonctions isocyanates, de préférence 2,5 à 3,1 fonctions isocyanates.

10. Plastifiant polymérisable selon la revendication 9, **caractérisé en ce que** le composé de type alcool est un monoalcool phénolique de formule générale (IV) : 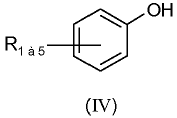 où R_{1 à 5} sont chacun indépendamment :
- halogène,
- alkyle,
- halogénoalkyle,
- cycloalkyle,
- aryle,
- alcoxy,
- arylalkyle,
- hétéroaryle,
- chaîne hydrocarbonée mono- ou polyinsaturée optionnellement substituée ;
et où R_{1 à 5} ne contiennent pas de fonction hydroxyle ni de fonction isocyanate ;
et où un au moins de R_{1 à 5} représente une chaîne polymère hydrocarbonée comprenant et/ou étant substituée par au moins un motif répétitif :
- alkyle,
- aryle,
- arylalkyle,
- arylcycloalkyle,
- arylhétérocycloalkyle,
- hétéroaryle,
- hétéroarylalkyle,
- hétéroarylcycloalkyle,
- hétéroarylhétérocycloalkyle,
- hydrocarbyle avec au moins une insaturation.

11. Plastifiant polymérisable selon la revendication 9, **caractérisé en ce que** le composé de type alcool est un monoalcool phénolique de formule générale (V) : 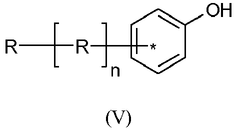 où chaque R représente un motif issu de la polymérisation de l'alpha-méthylstyrène.

12. Plastifiant polymérisable selon l'une quelconque des revendications 9 à 11, **caractérisé en ce que** le composé de type alcool est une résine de coumarone substituée par un phénol à une extrémité ou une résine alpha-méthylstyrène substituée par un phénol à une extrémité.

13. Composition de résine polyuréthane liquide, de préférence monocomposante stable, comprenant au moins un plastifiant polymérisable selon l'une quelconque des revendications 1 à 12, un prépolymère, et éventuellement un solvant, un catalyseur, des charges liquides et/ou des charges solides.

14. Méthode pour diminuer la quantité de solvant dans des compositions de résine polyuréthane liquides classiques, notamment dans les compositions de résine polyuréthane liquides monocomposantes, comprenant l'ajout du plastifiant polymérisable selon l'une quelconque des revendications 1 à 12, à une composition de polyuréthane en remplacement d'une partie ou de la totalité du solvant.

15. Méthode pour diminuer la quantité de plastifiant exogène dans des compositions de résine polyuréthane classiques, notamment dans les compositions de résine polyuréthane liquides monocomposantes, comprenant l'ajout du plastifiant polymérisable selon l'une quelconque des revendications 1 à 12, à une composition de polyuréthane en remplacement d'une partie ou de la totalité du plastifiant exogène.

## Patentansprüche

1. Polymerisierbarer Weichmacher, welcher eine Kohlenwasserstoffkette aufweist, an welcher ein einziges Ende mehr als eine Isocyanatfunktion trägt, wobei die Kohlenwasserstoffkette einen aromatischen Ring und/oder einen aliphatischen Ring umfasst und/oder mit ihm substituiert ist, und/oder wobei die Kohlenwasserstoffkette mit wenigstens zwei Kohlenwasserstoffketten substituiert ist, die eine Unsättigung aufweisen können, und wobei die Anzahl der Isocyanatfunktionen streng größer als 1 ist, bevorzugt größer als 1,2, noch stärker bevorzugt größer als 1,5 und kleiner gleich 2,2.

2. Polymerisierbarer Weichmacher nach Anspruch 1, welcher ein Molekulargewicht zwischen 600 und 3 000 g/mol aufweist, bevorzugt zwischen 700 und 2 500 g/mol und noch stärker bevorzugt zwischen 800 und 2 000 g/mol.

3. Polymerisierbarer Weichmacher nach Anspruch 1 oder 2, welcher eine Viskosität zwischen 400 und 14 000 Centipoise aufweist, bevorzugt zwischen 1 300 und 13 000 Centipoise und noch stärker bevorzugt zwischen 2 000 und 12 000 Centipoise.

4. Polymerisierbarer Weichmacher nach einem der Ansprüche 1 bis 3, welcher die folgende allgemeine Formel (I) aufweist: 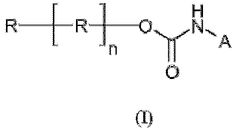 wobei jedes R unabhängig eine sich wiederholende Einheit ist, die keine Hydroxylfunktion und keine Isocyanatfunktion umfasst und die gewählt ist aus der Gruppe, umfassend:
- Alkyl,
- Cycloalkyl,
- Aryl,
- Heteroaryl,
- Arylcycloalkyl,
- Arylheterocycloalkyl,
- Heteroarylalkyl,
- Heteroarylcycloalkyl,
- einfach oder mehrfach ungesättigtes Hydrocarbyl;
und wobei n zwischen 2 und 50 liegt, bevorzugt zwischen 3 und 30 und noch stärker bevorzugt zwischen 5 und 25;
und wobei A eine Gruppe ist, umfassend eine Anzahl von Isocyanatfunktionen streng größer 1, bevorzugt größer 1,2, noch stärker bevorzugt größer 1,5 und kleiner gleich 2,2;
unter der Bedingung, dass die Formel (I) entweder wenigstens einen aromatischen oder aliphatischen Ring in wenigstens einer Einheit R umfasst, oder wenigstens zwei mit einer Alkylkette substituierte Alkyleinheiten R umfasst.

5. Polymerisierbarer Weichmacher nach einem der Ansprüche 1 bis 4, welcher die folgende allgemeine Formel (II) aufweist: 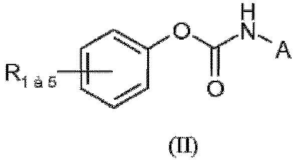 wobei die Substituenten R_{1 bis 5}, jeweils voneinander unabhängig, sind:
- Halogen,
- Alkyl,
- Halogenalkyl,
- Cycloalkyl,
- Aryl,
- Alcoxy,
- Arylalkyl,
- Heteroaryl,
- einfach oder mehrfach ungesättigte Wassersstoffkette;
und wobei R_{1 bis 5} weder eine Hydroxylfunktion noch eine Isocyanatfunktion aufweisen;
und wobei wenigstens einer von R_{1 bis 5} für eine Kohlenwasserstoffpolymerkette steht, welche wenigstens eine sich wiederholende Einheit umfasst und/oder mit dieser substituiert ist:
- Alkyl,
- Aryl,
- Arylalkyl,
- Arylcycloalkyl,
- Arylheterocycloalkyl,
- Heteroaryl,
- Heteroarylalkyl,
- Heteroarylcycloalkyl,
- Heteroarylheterocycloalkyl,
- Hydrocarbyl mit wenigstens einer Unsättigung;
und wobei A eine Gruppe ist, umfassend eine Anzahl von Isocyanatfunktionen streng größer 1, bevorzugt größer 1,2, noch stärker bevorzugt größer 1,5 und kleiner gleich 2,2.

6. Polymerisierbarer Weichmacher nach Anspruch 5,
**dadurch gekennzeichnet, dass** die Kohlenwasserstoffpolymerkette die folgende allgemeine Formel (IIIa) oder (IIIb) aufweist: 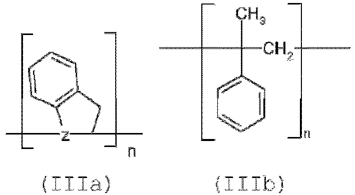 wobei Z ein Kohlenstoff- und/oder Sauerstoffatom ist; und wobei n zwischen 2 und 50 liegt, bevorzugt zwischen 3 und 30 und noch stärker bevorzugt zwischen 5 und 25.

7. Polymerisierbarer Weichmacher nach Anspruch 6,
**dadurch gekennzeichnet, dass** die Kohlenwasserstoffpolymerkette die allgemeine Formel (IIIb) aufweist, wobei n zwischen 2 und 50 liegt, bevorzugt zwischen 3 und 30 und noch stärker bevorzugt zwischen 5 und 25, und wobei A eine Polymerkette des MDI-Polymers ist, welche die folgende Formel (A1) aufweist: 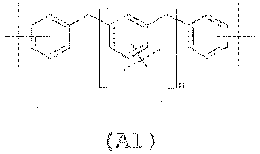 und wobei A eine Anzahl von Isocyanatfunktionen umfasst, die streng größer als 1 und kleiner gleich 2,2 ist.

8. Polymerisierbarer Weichmacher nach Anspruch 6,
**dadurch gekennzeichnet, dass** die Kohlenwasserstoffpolymerkette die allgemeine Formel (IIIb) aufweist, wobei n zwischen 2 und 50 liegt, bevorzugt zwischen 3 und 30 und noch stärker bevorzugt zwischen 5 und 25, und wobei A ein Radikal ist, welches die folgende Formel (A2) aufweist, oder ein dem TDI-Trimer verwandtes Radikal ist: 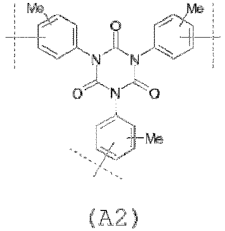 und wobei A eine Anzahl von Isocyanatfunktionen umfasst, die streng größer als 1 und kleiner gleich 2,2 ist.

9. Polymerisierbarer Weichmacher nach einem der Ansprüche 1 bis 8,
**dadurch gekennzeichnet, dass** er erhalten werden kann durch eine Reaktion zwischen:
- einer Zusammensetzung vom Alkoholtyp, welche eine Kohlenwasserstoffkette aufweist, die einen aromatischen Ring und/oder einen aliphatischen Ring umfasst und/oder mit ihm substituiert ist, und/oder wobei die Kohlenwasserstoffkette der Zusammensetzung vom Alkoholtyp mit wenigstens zwei Kohlenwasserstoffketten substituiert ist, die eine Unsättigung aufweisen können, und wobei die Zusammensetzung vom Alkoholtyp eine Anzahl -OH umfasst, die zwischen 0,8% und 2,5% liegt, bevorzugt zwischen 1,3% und 2,4% und noch stärker bevorzugt zwischen 1,4% und 1,7% Gewichtsprozent im Verhältnis zum Gewicht der Zusammensetzung, und
- einem Polyisocyanat, welches 2,1 bis 3,2 Isocyanatfunktionen, bevorzugt 2,5 bis 3,1 Isocyanatfunktionen, umfasst.

10. Polymerisierbarer Weichmacher nach Anspruch 9,
**dadurch gekennzeichnet, dass** die Zusammensetzung vom Alkoholtyp ein einwertiger phenolischer Alkohol ist, der die folgende allgemeine Formel (IV) aufweist: 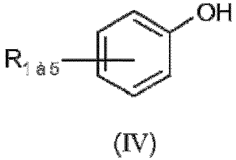 wobei R_{1 bis 5}, jeweils voneinander unabhängig, sind:
- Halogen,
- Alkyl,
- Halogenalkyl,
- Cycloalkyl,
- Aryl,
- Alcoxy,
- Arylalkyl,
- Heteroaryl,
- einfach oder mehrfach ungesättigte Wassersstoffkette, optional substituiert;
und wobei R_{1 bis 5} weder eine Hydroxylfunktion noch eine Isocyanatfunktion aufweisen;
und wobei wenigstens einer von R_{1 bis 5} für eine Kohlenwasserstoffpolymerkette steht, welche wenigstens eine sich wiederholende Einheit umfasst und/oder mit diesen substituiert ist:
- Alkyl,
- Aryl,
- Arylalkyl,
- Arylcycloalkyl,
- Arylheterocycloalkyl,
- Heteroaryl,
- Heteroarylalkyl,
- Heteroarylcycloalkyl,
- Heteroarylheterocycloalkyl,
- Hydrocarbyl mit wenigstens einer Unsättigung.

11. Polymerisierbarer Weichmacher nach Anspruch 9,
**dadurch gekennzeichnet, dass** die Zusammensetzung vom Alkoholtyp ein einwertiger phenolischer Alkohol ist, der die folgende allgemeine Formel (V) aufweist: 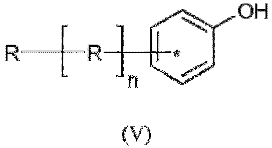 wobei jedes R für eine Einheit aus der Polymerisation von Alphamethylstyrol steht.

12. Polymerisierbarer Weichmacher nach einem der Ansprüche 9 bis 11,
**dadurch gekennzeichnet, dass** die Zusammensetzung vom Alkoholtyp ein an einem Ende mit einem Phenol substituiertes Cumaronharz oder ein an einem Ende mit einem Phenol substituiertes Alphamethylstyrol-Harz ist.

13. Flüssige Polyurethanharz-Zusammensetzung, bevorzugt eine stabile Einkomponentenzusammensetzung, welche wenigstens einen polymerisierbaren Weichmacher nach einem der Ansprüche 1 bis 12, ein Prepolymer und möglicherweise ein Lösungsmittel, einen Katalysator, flüssige Füllstoffe und/oder feste Füllstoffe umfasst.

14. Verfahren zur Verringerung der Lösungsmittelmenge in klassischen flüssigen Polyurethanharz-Zusammensetzungen, insbesondere in flüssigen Polyurethanharz-Einkomponentenzusammensetzungen, welche den Zusatz des polymerisierbaren Weichmachers nach einem der Ansprüche 1 bis 12 zu einer Polyurethanzusammensetzung als teilweiser oder vollständiger Ersatz des Lösungsmittels umfassen.

15. Verfahren zur Verringerung der Menge exogenen Weichmachers in klassischen Polyurethanharz-Zusammensetzungen, insbesondere in flüssigen Polyurethanharz-Einkomponentenzusammensetzungen, welche den Zusatz eines polymerisierbaren Weichmachers nach einem der Ansprüche 1 bis 12 zu einer Polyurethanzusammensetzung als teilweiser oder vollständiger Ersatz des exogenen Weichmachers umfassen.

## Claims

1. A polymerizable plasticizer containing a hydrocarbon chain, only one end of which bears more than one isocyanate function, said hydrocarbon chain comprising and/or being substituted with an aromatic ring and/or an aliphatic ring and/or said hydrocarbon chain being substituted with at least two hydrocarbon chains that may comprise an unsaturation, and the number of isocyanate functions being strictly greater than 1, preferably greater than 1.2, more preferentially greater than 1.5 and less than or equal to 2.2.

2. The polymerizable plasticizer according to claim 1, having a molecular weight between 600 and 3,000 g/mol, preferably between 700 and 2,500 g/mol and even more preferentially between 800 and 2,000 g/mol.

3. The polymerizable plasticizer according to claim 1 or 2, having a viscosity between 400 and 14,000 centipoises, preferably between 1,300 and 13,000 centipoises and even more preferentially between 2,000 and 12,000 centipoises.

4. The polymerizable plasticizer according to any one of claims 1 to 3, having general formula (I): 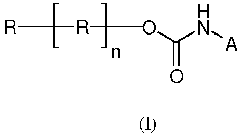 wherein each R independently represents a repeating unit which does not comprise any hydroxyl functions or any isocyanate functions and which is chosen from the group comprising:
- alkyl,
- cycloalkyl,
- aryl,
- heteroaryl,
- arylcycloalkyl,
- arylheterocycloalkyl,
- heteroarylalkyl,
- heteroarylcycloalkyl,
- monounsaturated or polyunsaturated hydrocarbyl;
and wherein n is between 2 and 50, preferably between 3 and 30 and even more preferentially between 5 and 25;
and wherein A is a group comprising a number of isocyanate functions strictly greater than 1, preferably greater than 1.2 and more preferentially greater than 1.5 and less than or equal to 2.2;
on condition that formula (I) comprises either at least one aromatic or aliphatic ring in at least one unit R or at least two alkyl units R substituted with an alkyl chain.

5. The polymerizable plasticizer according to any one of claims 1 to 4, having general formula (II) 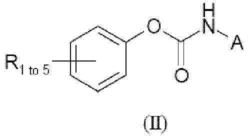 wherein substituents R_{1 to 5} are each independently:
- halogen,
- alkyl,
- haloalkyl,
- cycloalkyl,
- aryl,
- alkoxy,
- arylalkyl,
- heteroaryl,
- monounsaturated or polyunsaturated hydrocarbon chain;
and wherein R_{1 to 5} do not contain any hydroxyl functions or any isocyanate functions;
and wherein at least one from among R_{1 to 5} represents a hydrocarbon polymer chain comprising and/or being substituted with at least one repeating unit from the following:
- alkyl,
- aryl,
- arylalkyl,
- arylcycloalkyl,
- arylheterocycloalkyl,
- heteroaryl,
- heteroarylalkyl,
- heteroarylcycloalkyl,
- heteroarylheterocycloalkyl,
- hydrocarbyl with at least one unsaturation;
and wherein A is a group comprising a number of isocyanate functions strictly greater than 1, preferably greater than 1.2 and more preferentially greater than 1.5 and less than or equal to 2.2.

6. The polymerizable plasticizer according to claim 5, **characterized in that** the hydrocarbon polymer chain corresponds to general formula (IIIa) or (IIIb): 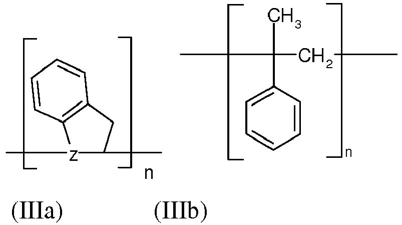 wherein Z is a carbon and/or oxygen atom; and wherein n is between 2 and 50, preferably between 3 and 30 and even more preferentially between 5 and 25.

7. The polymerizable plasticizer according to claim 6, **characterized in that** the hydrocarbon polymer chain corresponds to general formula (IIIb) wherein n is between 2 and 50, preferably between 3 and 30 and even more preferentially between 5 and 25, and wherein A is a polymer chain originating from a polymeric MDI corresponding to formula (A1): 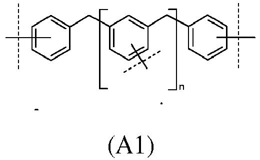 and wherein A comprises a number of isocyanate functions strictly greater than 1 and less than or equal to 2.2.

8. The polymerizable plasticizer according to claim 6, **characterized in that** the hydrocarbon polymer chain corresponds to general formula (IIIb), wherein n is between 2 and 50, preferably between 3 and 30 and even more preferentially between 5 and 25, and wherein A is a radical corresponding to formula (A2) or to a related radical of a TDI trimer: 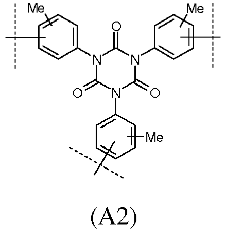 and wherein A comprises a number of isocyanate functions strictly greater than 1 and less than or equal to 2.2.

9. The polymerizable plasticizer according to any one of claims 1 to 8, **characterized in that** it may be obtained by reaction between:
- an alcohol type compound consisting of a hydrocarbon chain comprising and/or being substituted with an aromatic ring and/or an aliphatic ring and/or the hydrocarbon chain of the alcohol type compound being substituted with at least two hydrocarbon chains that may comprise an unsaturation and wherein the alcohol type compound has an -OH number between 0.8% and 2.5%, preferably between 1.3% and 2.4% and even more preferentially between 1.4% and 1.7% by weight relative to the weight of said compound; and
- a polyisocyanate comprising 2.1 to 3.2 isocyanate functions and preferably 2.5 to 3.1 isocyanate functions.

10. The polymerizable plasticizer according to claim 9, **characterized in that** the alcohol type compound is a phenolic monoalcohol of general formula (IV): 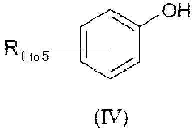 wherein R_{1 to 5} are each independently:
- halogen,
- alkyl,
- haloalkyl,
- cycloalkyl,
- aryl,
- alkoxy,
- arylalkyl,
- heteroaryl,
- optionally substituted monounsaturated or polyunsaturated hydrocarbon chain;
and wherein R_{1 to 5} do not contain any hydroxyl functions or any isocyanate functions;
and wherein at least one from among R_{1 to 5} represents a hydrocarbon polymer chain comprising and/or being substituted with at least one repeating unit:
- alkyl,
- aryl,
- arylalkyl,
- arylcycloalkyl,
- arylheterocycloalkyl,
- heteroaryl,
- heteroarylalkyl,
- heteroarylcycloalkyl,
- heteroarylheterocycloalkyl,
- hydrocarbyl with at least one unsaturation.

11. The polymerizable plasticizer according to claim 9, **characterized in that** the alcohol type compound is a phenolic monoalcohol of general formula (V): 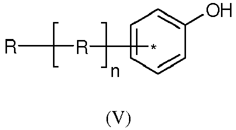 wherein each R represents a unit derived from the polymerization of α-methylstyrene.

12. The polymerizable plasticizer according to any one of claims 9 to 11, **characterized in that** the alcohol type compound is a coumarone resin substituted with a phenol at one end or an α-methylstyrene resin substituted with a phenol at one end.

13. A liquid polyurethane resin composition, preferably a stable one-component composition, comprising at least one polymerizable plasticizer according to any one of claims 1 to 12, a prepolymer, and optionally a solvent, a catalyst, liquid fillers and/or solid fillers.

14. A method for reducing the amount of solvent in standard liquid polyurethane resin compositions, especially in one-component liquid polyurethane resin compositions, comprising the addition of the polymerizable plasticizer according to any one of claims 1 to 12 to a polyurethane composition in replacement for some or all of the solvent.

15. A method for reducing the amount of exogenous plasticizer in standard polyurethane resin compositions, especially in one-component liquid polyurethane resin compositions, comprising the addition of the polymerizable plasticizer according to any one of claims 1 to 12 to a polyurethane composition in replacement for some or all of the exogenous plasticizer.
